(19) Europäisches Patentamt
European Patent Office
Office européen des brevets

(11) Veröffentlichungsnummer: **0 028 331**
**B2**

# (12) NEUE EUROPÄISCHE PATENTSCHRIFT

(45) Veröffentlichungstag der neuen Patentschrift: **20.05.87**

(51) Int. Cl.⁴: **C 07 C 125/063**, C 07 C 125/07, C 07 C 125/077, C 07 D 307/82

(21) Anmeldenummer: **80106249.8**

(22) Anmeldetag: **15.10.80**

## (54) Verfahren zur Herstellung von Urethanen.

(30) Priorität: **27.10.79 DE 2943550**

(43) Veröffentlichungstag der Anmeldung:
**13.05.81 Patentblatt 81/19**

(45) Bekanntmachung des Hinweises auf die Patenterteilung:
**15.12.82 Patentblatt 82/50**

(45) Bekanntmachung des Hinweises auf die Entscheidung über den Einspruch:
**20.05.87 Patentblatt 87/21**

(84) Benannte Vertragsstaaten:
**AT BE CH DE FR GB IT LI NL SE**

(56) Entgegenhaltungen:
**DE-A-2 258 454**
**DE-C-753 127**
**US-A-2 409 712**
**US-A-2 677 698**
**US-A-2 806 051**

**Journal of the American Chemical Society Tomb 76, Seiten 3977-3978 1954**

(73) Patentinhaber: **BAYER AG, Konzernverwaltung RP Patentabteilung, D-5090 Leverkusen 1 Bayerwerk (DE)**

(72) Erfinder: **Heitkämper, Peter, Dr., Fliederweg 14, D-4047 Dormagen 11 (DE)**
Erfinder: **Fauss, Rudolf, Dr., Gerstenkamp 10, D-5000 Koeln 80 (DE)**
Erfinder: **Findeisen, Kurt, Dr., In der Follmühle 10, D-5068 Odenthal 2 (DE)**
Erfinder: **Penninger, Stefan, Dr., Pommernallee 5, D-4047 Dormagen (DE)**
Erfinder: **Scholl, Hans-Joachim, Dr., Rudolf Sohm-Strasse 28, D-5000 Koeln 80 (DE)**

EP 0 028 331 B2

LIBER, STOCKHOLM 1987

**Beschreibung**

Verfahren zur Herstellung von Urethanen

Die vorliegende Erfindung betrifft ein verbessertes Verfahren zur Herstellung N,O-disubstituierten Urethanen durch Umsetzung von substituierten Harnstoffen mit Alkoholen.

Urethane entstehen bekanntlich u.a. durch Umsetzung von organischen Isocyanaten mit Alkoholen. Diese Umsetzung ist reversibel, d.h. Urethane können thermisch in das ihnen zugrundeliegende Isocyanat und den ihnen zugrundeliegenden Alkohol gespalten werden (vgl. z.B. US-A-24 09 712). Thermisch in Isocyanete spaltbare Urethane sind daher interessante Ausgangsmaterialien zur Herstellung dieser Isocyanate, die bislang weltweit in größtem Umfang durch Umsetzung von primären Aminen mit Phosgen hergestellt werden. Die phosgenfreie Herstellung von Urethanen und deren anschließende thermische Spaltung stellt eine interessante Alternative zu der genannten großtechnischen Isocyanetherstellung dar. Eine Methode zur phosgenfreien Herstellung von Urethenen besteht in der Umsetzung von substituierten Harnstoffen mit Alkoholen (vgl. z.B. US-A-24 09 712). Diese Methode des Standes der Technik ist jedoch mit dem Nachteil behaftet, daß die Urethane nur in unzureichender Ausbeute entstehen, da, wie der in der genannten Literaturstelle offenbarten Reaktionsgleichung zu entnehmen, stets das dem Harnstoff-Substituenten entsprechende primäre Amin als Nebenprodukt anfällt. Die DE-C-753 127 betrifft die Herstellung von N-unsubstituierten Urethanen (Carbaminsäureestern) und kann daher keinerlei Hinweis auf ein Verfahren zur Herstellung N-substituierten Urethanen vermitteln, die als Zwischenprodukte zur Phosgen-freien Herstellung von Isocyanaten eingesetzt werden könnten. Die DE-A-2 258 454 betrifft die Herstellung von N-monosubstituierten Carbamaten durch Umsetzung von trisubstituierten Harnstoffen rnit Hydroxylverbindungen in Gegenwart von gasförmigem Chlorwasserstoff. Auch diese Vorveröffentlichung kann keinerlei Hinweis auf das nachstehend näher beschriebene erfindungsgemäße Verfahren vermitteln, bei welchem andersartige Ausgangsmaterialien eingesetzt werden.

Es war die Aufgabe der vorliegenden Erfindung, das aus US-A-2 409 712 bekannte Verfahren zur Herstellung von N,O-disubstiuierten Urethanen durch Umsetzung von substituierten Harnstoffen mit Alkoholen so zu verbessern, daß die Bildung von unerwünschten Nebenprodukten, insbesondere von primären Aminen weitgehend vermieden wird, und die Urethane in verbesserter Ausbeute erhalten werden.

Überraschenderweise wurde nunmehr gefunden, daß diese Aufgabe durch die gleichzeitige Mitverwendung von nachstehend näher beschriebenen Reaktionspartner gelöst werden kann.

Gegenstand der Erfindung ist ein Verfahren zur Herstellung von Urethanen der allgemeinen Formel

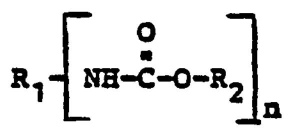

in welcher

$R_1$ fü einen gegebenenfalls substituierten aliphatischen Kohlenwasserstoff rest mit 1-18 Kohlenstoffatomen, einen gegebenenfalls substituierten cycloaliphatischen Kohlenwasserstoffrest mit 3 bis 18 Kohlenstoffatomen, einen gegebenenfalls substituierten aromatischen Kohlenwasserstoffrest mit 6-15 Kohlenstoffatomen, einen gegebenenfalls substituierten araliphatischen Kohlenwasserstoffrest mit 7 bis 14 Kohlenstoffatomen, oder für einen gegebenenfalls substituierten 5-oder 6-gliedrigen heterocyclischen Rest steht, der zusätzlich noch mit einem Benzolring anelliert sein kann.

$R_2$ für einen gegebenenfalls substituierten Alkylrest mit 1 bis 20 Kohlenstoffatornen, einen gegebenenfalls substituierten Cycloalkylrest mit 3 bis 16 Kohlenstoffatomen und einen gegebenenfalls substituierten Aralkylrest mit 7 bis 14 Kohlenstoffatomen und

n eine ganze Zahl von 1 bis 3 bedeuten,

wobei im Falle von n = 2 oder 3 zwischen 2 mit dern Rest $R_1$ verknüpften Urethangruppen mindestens 2 Kohlenstoffatome angeordnet sind, durch Umsetzung als Ausgangsmaterialien von

a) gegebenenfalls Urethan- oder Aminoendgruppen aufweisenden N-mono- oder N,N'-disubstituierten Harnstoffen oder linearen Polyharnstoffen eines rnaxirnalen Molekulargewichts von 2 000, vorzugsweise maximal 700, deren Harnstoff-, Urethan- bzw. Aminogruppen über Kohlenwasserstoffreste miteinander verknüpft sind, bzw. deren Harnstoffgruppen mit Kohlenwasserstoffresten substiuiert sind, die die Bedeutung von $R_1$ haben, deren gegebenenfalls vorliegende endständige Urethangruppen am Sauerstoffatom eines Substituenten aufweisen, dessen Bedeutung der Definition von $R_2$ entspricht, und deren Gesamtgehalt an Harnstoffgruppen -NH-CO-NH- und Urethangruppen -NH-CO-O- einem Gehalt an für beide Gruppen charakteristischen Struktureinheiten -NH-CO zwischen 5 und 58 Gew.-%. vorzugsweise zwischen 10 und 58 % entspricht, mit

b) Alkoholen der Formel $R_2$-OH

in welcher $R_2$ die bereits genannte Bedeutung hat, im Temperaturbereich von 120 bis 350°C, dadurch gekennzeichnet, daß man als weitere Reaktionspartner

c) Urethane der Formel

$R_3$-O-CO-$NH_2$

in welcher,

$R_3$ entweder der Bedeutung von $R_2$ entspricht, wobei $R_2$ und $R_3$ gleiche oder verschiedene Reste bedeuten, oder für einen gegebenenfalls Chlor- oder $C_1$-$C_4$-Alkyl-substituierten, aromatischen Kohlenwasserstoff rest mit insgesamt 6-15-Kohlenstoffatomen steht, und/oder

d) Harnstoff und/oder Polyurete der Formel

$H_2N$-$(CO$-$NH)_m$-$H$

in einer Menge von mindestens 10 gew.-% der Komponenten c) und/oder d) benzogen auf die Komponente a) verwendet,

wobei m für eine ganze Zahl von 1 bis 4 steht.

Als Substituenten für die aliphatischen oder cycloaliphatischen Reste $R_1$ bzw. $R_2$ kommen beispielsweise $C_6$-$C_{10}$-Aroxy., $C_1$-$C_6$-Alkoxy, $C_1$-$C_6$-Alkoxy-$C_2$-$C_4$-alkoxy-, $C_1$-$C_6$-Acyl., $C_1$-$C_6$-Alkylmercapto-, $C_6$-$C_{10}$-Arylmercapto-, $C_1$-$C_{12}$-Alkyl-carbonyl-, Bis-($C_1$-$C_8$-Alkyl)-amino, $C_1$-$C_6$-Acylamino-, Nitro-, Cyano- oder Rhodanoreste in Betracht.

Als Substituenten für die aromatischen oder araliphatischen Reste $R_1$ bzw. $R_2$ kommen neben diesen Substituenten beispielsweise auch $C_1$-$C_{12}$-Alkyl-, $C_1$-$C_{12}$-Alkylsulfonyl, $C_6$-$C_{10}$-Arylsulfonyl-, $C_1$-$C_{12}$-Alkylsulfonsäureester-, oder Sulfonamidreste in Betracht.

Bevorzugte erfindungsgemäße Verfahrensprodukte sind jedoch solche der gennanten allgemeinen Formel, für welche

$R_1$ für einen aliphatischen Kohlenwasserstoff rest mit 3 bis 18 Kohlenstoffatomen, einen cycloaliphatischen Kohlenwasserstoffrest mit 6 bis 15 Kohlenstoffatomen oder einen gegebenenfalls Methyl-, Methoxy- oder Chlor-substituierten und gegebenenfalls Methylenbrücken aufweisenden aromatischen Kohlenwasserstoffrest mit 6 bis 15 Kohlenstoffatomen steht,

$R_2$ für einen $C_1$-$C_4$-Alkoxy- oder $C_1$-$C_4$-Alkoxy-$C_2$-$C_4$-alkoxy-substituierten oder unsubstituierten aliphatischen Kohlenwasserstoffrest mit 1 bit 18, insbesondere 1 bis 4 Kohlenstoffatomen steht, wie er durch Entfernung der Hydroxylgruppe aus einem einerwertigen unsubstituierten primären oder sekundären aliphatischen Alkohol erhalten wird oder für einen Cyclohexyl- oder 2-Phenyl-ethyl-Rest steht und

n 1 oder 2 bedeutet.

Beim erfindungsgemäßen Verfahren werden folgende Ausgangsmaterialien eingesetzt:

a) die bereits definierten substituierten Harnstoffe.

Typische Beispiele geeigneter substituierter Harnstoffe sind Methyl-, Ethyl-, Isobutyl-, Oktadecyl-, Phenyl-, Cyclohexyl-, oder Benzylharnstoff, N,N'-Dimethylharnstoff, N,N'-(3,3'-Diisobutylharnstoff, N,N'-Diphenylharnstoff, N,N'-(3,3'-Di-dibenzofuranyl)-harnstoff, N,N'-Di-(4-chlorphenyl)-harnstoff, N,N'-Di-(4-methoxyphenyl)-harnstoff, N,N'-Dicyclohexylharnstoff oder Verbindungen der Formeln

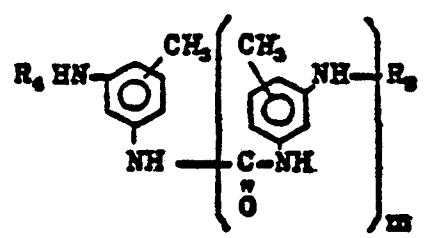

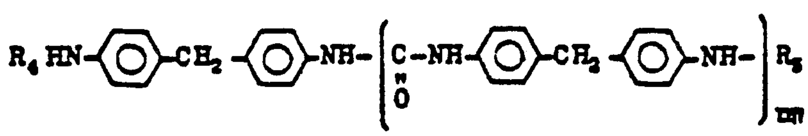

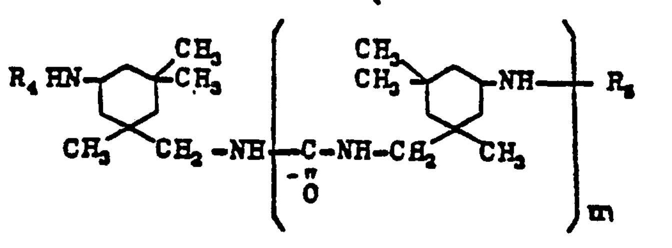

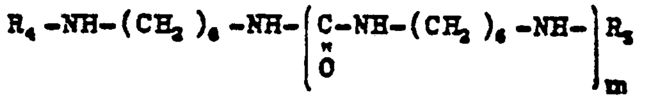

wobei m eine ganze oder gebrochene (bei statistischen Gemischen) Zahl von 1 bis 10 ist, $R_4$ und $R_5$ für gleiche oder verschiedene Reste stehen und jeweils H, $COOR_2$, $CONH_2$, $CONHR_6$ bedeuten, wobei $R_6$ für einen einwertigen Rest der bei der Definition von $R_1$ genannten Art steht.

Bei den substituierten Harnstoffen a) handelt es sich um bekannte Verbindungen deren Herstellung in der chemischen Fachliteratur beschrieben ist (vgl. z.B. D. F. Kutepow, Russ. Chem. Rev. 31, 633 (1962) oder H. Rinke, Houben-Weyl XIV/2, 185 ff). Erfindungsgemäß geeignete substituierte Harnstoffe a) sind auch die bei den Verfahren der US-Patentschriften 24 09 712 und 28 05 051 als Nebenprodukte bei der Urethansynthese aus

Harnstoff, Amin und Alkohol anfallenden Verbindungen. Die beispielhaft genannten N,N'-disubstituierten Monoharnstoffe und die Bis-Harnstoffe werden bevorzugt eingesetzt.

b) Alkohole der Formel

$R_2$—OH

in welcher

$R_2$ die bereits genannte Bedeutung hat.

Beispiele geeigneter Alkohole sind Methanol, Ethanol, Propanol, iso-Propanol, Butanol, iso-Butanol, Pentanol, iso-Pentanol, Hexanol, iso-Hexanol, Heptanol, iso-Heptanol, Octanol, iso-Octanol, Nonanol, iso-Nonanol, Decanol, iso-Decanol, Dodecanol, 2-Ethylhexanol, β-Chlorethanol, 2-Ethylbutanol, Hexadecanol, Octadecanol, Fettalkohol-Gemische, 2-Methoxyethanol, 2-Ethoxyethanol, 2-Propoxyethanol, 2-Butoxyethanol, 2-(2-Methoxyethoxy)-ethanol, 2-(2-Ethoxyethoxy)-ethanol, 2-(2-Butoxyethoxy)-ethanol, Cyclopentanol, Cyclohexanol. Methylcyclohexanol (und Gemisch), Cyclohexamethanol, 3,3,5-Tri-methylcyclohexanol, 4-tert.-Butylcyclohexanol, 2-Hydroxydecalin, Borneol, Isoborneol, 1-(2-Hydroxy-ethoxy)-4-nitrobenzol, Benzylalkohol, 2-Phenylethanol, 2-(Methoxyphenoxy)-ethanol (Gemisch), 1-Phenylethanol, 3-Phenyl-1-propanol, 4-Methoxybenzylalkohol.

Besonders bevorzugte Alkohole sind Methanol, Ethanol, n-Propanol, iso-Propanol, n-Butanol, iso-Butanol, Cyclohexanol, n-Hexanol, 2-Ethylhexanol, β-Phenylethanol, Glykolmonomethylether, Glykolmonobutylether oder Diglykolmonomethylether.

Erfindungswesentlich ist nun die gleichzeitige Mitverwendung von Reaktionspartnern c) und/oder d).

Bei den Reaktionspartnern c) handelt es sich um N-unsubstituierte Urethane der Formel

$R_3$-O-CO-$NH_2$

in welcher

$R_3$ die bereits genannte Bedeutung hat.

Typische Beispiele geeigneter N-unsubstituierter Urethane sind Methyl-, Ethyl-, n-Propyl-, iso-Propyl-, n-Butyl-, iso-Butyl-, Cyclohexyl oder Benzyl-carbamat, bzw. die sich von den oben beispielhaft genannten Alkoholen ableitenden N-unsubstituierten Urethane oder auch Phenyl-4-Chlorphenyl-, 4-Methylphenyl-oder 1-Naphthylcarbamat.

Bei den Reaktionspartnern d) handeltes sich um Harnstoff oder Polyurete, insbesondere Biuret, Triuret oder Tetrauret, d.h. um Verbindungen der Formel

$H_2N$-$(CO$-$NH)_m$-H

in welcher

m für eine ganze Zahl von 1 bis 4 steht, bzw. Gemische derartiger Verbindungen.

Bei der Durchführung des erfindungsgemäßen Verfahrens wird die Alkoholkomponente b), bezogen auf die in den Komponenten a), c) und d) vorliegenden Teil einer Harnstoff - oder einer Urethangruppe bildenden Carbonylgruppen, in mindestens stöchiometrischer Menge eingesetzt, vorzugsweise wird jedoch eine überstöchiometrische Menge der Alkoholkomponente b) verwendet, so das daß Äquivalentverhältnis von Hydroxylgruppen zu Carbonylgruppen zwischen 1 : 1 und 10 : 1, insbesondere zwischen 1 : 1 und 5 : 1 liegt. Bei dieser Berechnung des Mengenverhältnisses ist der in der Komponente c) in Form einer Urethangruppe gebundene Alkohol und der in der Komponente a) gegebenenfalls in Form einer Urethangruppe gebundene Alkohol dann zu berücksichtigen, wenn dieser Alkohol der Alkoholkomponente b) entspricht. Falls es sich bei diesen chemisch gebundenen Alkoholen um von der Alkoholkomponente b) verschiedene Alkohole handelt, die bei der Umsetzung verdrängt werden, gehen diese Alkohole, nicht in die Berechnung der Mengenverhältnisse ein. Die N-unsubstituierten Urethane c) werden bei der Durchführung des erfindungsgemäßen Verfahrens in einer Menge von 0 bis 300, vorzugsweise 0 bis 150 Gew.-%, bezogen auf die substituierten Harnstoffe a) und die Harnstoffe und/oder Polyurete d) in einer Menge von 0 bis 100, vorzugsweise 0 bis 70 Gew.-%, bezogen auf substituierte Harnstoffe a) verwendet, mit der Einschränkung, daß die Gesamtmenge der Komponente c) und d), bezogen auf die Menge der Komponente a), mindestens 10 Gew,-%, vorzugsweise mindestens 15 Gew.-% beträgt.

Das erfindungsgemäße Verfahren wird vorzugsweise in Anwesenheit geeigneter Katalysatoren e) durchgeführt. Geeignete Katalysatoren sind alle beliebigen Verbindungen, die einen katalytischen Einfluß auf die Veresterungsgeschwindigkeit von Carbonsäuren ausüben. Für das erfindungsgemäße Verfahren besonders gut geeignete Katalysatoren sind (i) unter den Reaktionsbedingungen inerte anorganische oder organische Basen, (ii) Lewis-Säuren und (iii) Salze bzw. Komplexverbindungen, insbesondere Chelate von Übergangsmetallen.

Beispiele geeigneter Katalysatoren der Gruppe (i) sind tert.-Amine wie Tri-n-propylamin, Triethylamin, Triisopentylamin, Diethylbenzylamin, N,N-Dimethyl-benzylamin, Hexahydrodimethylanilin, N -Ethylpiperazin, Diethyl (2-methoxypropyl)-amin, 2-(Diethylaminoethyl)-phenylether, N-(2-Diethylami-noethyl)-benzamid, N-(2-Diethylaminoethyl)-propionamid, 1,4-Diaza-(2,2,2)-bicyclooctan, N,N-Dimethyl-4-aminopyridin, 1-Azabicycloheptane, 1-Azabicyclooctane, gesättigte polyheterocyclische Amine, wie 3-Methylconidin, 1-Azabicyclo-(3,2,1)-octan, Pyrrolizidine und Chinuclidine, anorganische Basen wie Berylliumhydroxid und Natrium-, Kalium-, Lithium-, Magnesium-, Barium- oder Calciumhydroxid, basische Alkalisalze wie Natriumcarbonat, Natriumsulfid, Kaliumcarbonat oder Trinatriumphosphat sowie Alkalisalze von Fettsäuren oder Sulfonsäuren.

Geeignete Katalysatoren (II) sind beispielsweise Lewis-Säuren wie Eisen-II-chlorid, Eisen-III-chlorid, Zinkchlorid, Zinn-II-chlorid, Zinn-IV-chlorid, Aluminium-chlorid, Zinkcyanid, Bortrifluorid oder

Bortrifluoridetherat.

Geeignete Katalysatoren der Gruppe (iii), sind beispielsweise Salze von Übergangsmetallen, soweit sie nicht bereits in die Gruppe (ii) fallen, sowie Komplexverbindungen insbesondere Chelate dieser Metalle wie Kobalt-, Mangan-, oder Bleinaphthenate, Eisenoleate oder -carbonyle, Acetylacetonate von Eisen, Nickel, Kobalt, Zink, Blei Aluminium, Mangan, Magnesium, Molybdän, Titan, Torium, Zirkon oder Vanadium, Bis-(dibenzoylmethan)-kupfer, Bis-(ethylacetoacetat)-kupfer, -eisen, Koordinationsverbindungen von Titan, Zirkon, Hafnium, Thorium und Mangan mit β-Diketonen, β-Ketoestern und β-Hydroxyaldehyden, Dibutylzinndilaurat, Dibutylzinndiacetat, Di-(2-Ethylhexyl)-zinnoxid, Dioctylzinnoxid, Zinnsalze von $C_1$-$C_{20}$-Carbonsäuren wie Zinn (II)-naphthenat, -hexoat, -calmitat, -stearat oder -di-methylvalerat, Acetate, Chloride, Sulfate oder Octoate des zwei- oder dreiwertigen Cobalts, des ein- oder zweiwertigen Kupfers oder des zweiwertigen Bleis.

Besonders gut geeignete Katalysatoren sind beispielsweise Zinkchlorid, Zinkacetat, Zinkoctoat, Zinkoxid, Zinkcyanid, Zinn-II-chlorid, Zinn-IV-chlorid, Dibutyl-zinndilaurat, Kobalttriacetat, Kobaltrichlorid, Kobalttrioctoat, Kupfer (II)-acetat, Kupfer-(I)-chlorid, Kupfer-(II)-sulfat, Bleiacetat oder Bleichlorid. Die Menge des jeweils eingesetzten Katalysators liegt im allgemeinen zwischen 1 ppm und 20 Gew.-%, bevorzugt zwischen 100 ppm und 5 Gew.-%, bezogen auf die Summe der Ausgangsmaterialien a), b), c) und d). Man wird in der Praxis selbstverständlich bestrebt bleiben, die Konzentration der Katalysatoren möglichst gering zu halten. Die optimale Konzentration hängt von der Art der Ausgangsmaterialien und der Aktivität des jeweiligen Katalysators ab und kann in einem einfachen Vorversuch bestimmt werden.

Die Durchführung des erfindungsgemäßen Verfahren kann sowohl unter Druck als auch drucklos erfolgen. Die Anwendung von Druck beispielsweise im Bereich von 1 bis 80 bar ist oft dann sinnvoll, wenn die Reaktionstemperatur oberhalb des Siedepunkts einer oder mehrerer der Ausgangsmaterialien liegt. Es ist auch möglich, a) und c) vorzuerhitzen und b) ohne Anwendung von Druck dem Gemisch so zu zudosieren, daß die Reaktionstemperatur unter gleichzeitigem Verbrauch des Alkohols erhalten bleibt. Bei Mitverwendung der Komponente d) wäre hierbei deren gleichzeitige Zugabe mit dem Alkhol zu den vorerhitzten Komponenten a) und gff. c) zweckmäßig. Das erfindungsgemäße Verfahren wird im Temperaturbereich von 120°C bis 350°C, vorzugsweise 130 bis 300 °C u nd insbeondere 140°C bis 250°C durchgeführt.

Das erfindungsgemäße Verfahren kann in Gegenwart oder auch in Abwesenheit von Lösungsmitteln durchgeführt werden. Geeignete Lösungsmittel sind beispielsweise unter den Verfahrensbedingungen inerte Lösungsmittel mit einem zwischen 100°C und 280°C, vorzugsweise zwischen 150°C und 250°C liegenden Siedepunkt. Beispiele geeigneter Lösungsmittel sind n-Nonan, n-Butylcyclohexan, Decahydronaphthalin, n-Undecan, n-Dodecan, n-Hexylcyclohexan, Dipenten, 1 -Dodecen, Isopropylbenzol, 1,3-Diethylbenzol, Inden, n-Butylbenzol, Tetralin, Chlorbenzol, 4-Chlortoluol, 1,2-Dichlorbenzol, 2,4-Di-chlortoluol, 1,2,4-Trichlorbenzol, 2-Chlor-4-isopropyl-1-methylbenzol, Anisol, Cyclohexylethylether, Diethylenglykoldimethylether, Benzylmethylethe r, 4-Methoxytoluol, Parachloranisol, Di-n-hexylether, Phenyl-npropylketon, Benzophenon, Acetophenon, Formamid, N,N-Dimethylformamid, N,N-Diethylformamid, N-Methylformamid, Dimethylacetamid, N-Methylpyrrolidon, Caprolactam, Phenol, substituierte Phenole, Sulfolan, Hexamethylphosphorsäuretriamid, Dimethylsulfoxid, Ethylenglykolmonomethyletheracetat, Di-n-propylcarbonat, Cyclohexylacetat, Diisobutylcarbonat, Diethylenglykolmonomethyletheracetat, Diisoamylcarbonat, 2-Ethylpyridin, N,N-Dimethyl-2-methylanilin, N, N-Dimethylanilin, N-Methyl-N-ethylanilin, N,N-Dimethyl-2-chloranilin, N,N-Diethylanilin, Chinolin, Nitrocyclohexan, Nitrobenzol, 2-Nitrotoluol, 2,4-Dimethyl-1 -nitrobenzol, Acetonitril, N-Capronitril, Benzonitril, Tolunitril, Diphenylether, Tetramethylharnstoff und Phenylacetonitril, besonders bevorzugt sind polare Lösungsmittel und deren Gemische. Ein besonders gut geeignetes Lösungsmittel ist ε-Caprolactam.

Die Mitverwendung derartiger Lösungsmittel ist oft, beispielsweise bei Verwendung eines hohen Überschusses an Alkohol (Komponente b) überflüssig. Insbesondere erübrigt ich die Verwendung derartiger Hilfslösungsmittel bei der Herstellung von Monourethanen (n = 1), d.h. bei der ausschließlichen Verwendung von N-mono- oder N,N'-disubstituierten Monoharnstoffen als Komponente a).

Zur Durchführung des erfindungsgemäßen Verfahrens werden im allgemeinen die genannten Ausgangsmaterialien während 1 bis 15, vorzugsweise 2 bis 12 Stunden auf die genannte Reaktionstemperatur gebracht, wobei dafür Sorge getragen wird, daß das sich bildende Ammoniak entweichen kann.

Im Falle der Verwendung eines Alkohols b), der nicht dem in der Komponente c) und gegebenenfalls der Komponente a) chemisch gebundene Alkohol einen tieferen Siedepunkt als die Alkoholkomponente b) aufweisen, so daß eine Verdrängung des gebundenen Alkohols durch die Alkoholkomponente b) möglich ist. Diese Verdrängungsreaktion des im Verfahrensprodukt unerwünschten Alkohols durch die Alkoholkomponente b) erfolgt im allgemeinen gleichzeitig mit der erfindungsgemäßen Reaktion. Sie kann jedoch dieser auch vorgeschaltet werden, beispielsweise dergestalt, daß man die Komponente c) zunächst mit einem Teil oder der Gesamtmenge der Alkoholkomponente b) auf eine geeignete, innerhalb der genannten Bereiche liegende Temperatur erhitzt und den sich dabei bildenden niedriger als die Alkoholkomponente b) siedenden Alkohol destillativ entfernt. Anschließend erfolgt dann die erfindungsgemäße Reaktion nach Zugabe der übrigen Ausgangsmaterialien. Im Falle der im Prinzip ebenfalls möglichen, jedoch weniger bevorzugten Verwendung von N-unsubstituierten Urethanen c), welche als O-Substituenten ein gegebenenfalls Chlor- oder $C_1$-$C_4$-Alkyl-substituiertes Phenol mit insgesamt 6 bis 15 Kohlenstoffatomen aufweisen, läuft eine analoge Verdrängungsreaktion auch dann ab, wenn das sich bildende Phenol nicht aus dem Ansatz enffernt wird.

Die Aufarbeitung der erfindungsgemäßen Verfahrenprodukte erfolgt in an an sich bekannter Weise insbesondere destillativ durch Abdestillieren leicht flüchtiger Bestandteile (Lösungsmittel und/oder leicht

flüchtige überflüssige Ausgangsmaterialien), gegebenenfalls nach vorhergehendem Abfiltrieren unlöslicher Bestandteile (beispielsweise unlösliche Katalysatoren), wobei die Verfahrensprodukte entweder als letzte Fraktion oder als Destillationsrückstand anfallen. Die zu erhaltenen Verfahrensprodukte können ohne weitere Reindarstellung direkt ihrem Verwendungszeck zugeführt werden. Dies bedeutet, daß die Verfahrensprodukte sofort thermisch in das ihnen zugrundeliegende Isocyanat und den ihnen zugrundeliegenden Alkohol gespalten werden können, wobei man sich der an sich bekannten Methoden des Standes der Technik bedient.

Die in den nachfolgenden Beispielen gemachten Prozentangaben beziehen sich auf Gewichtsprozente.

**Beispiel 1**

Eine Füllkörperkolonne aus Stahl (Nennweite 50 mm) mit einem Schlangenkühler als Kopfkondensator wird mit Füllringen (4 mm) aus Stahlmaschendrahtgewebe ca. 1 m hoch gefüllt. Oberhalb des Kopfkondensators befindet sich ein Ventil zur Entnahme von Gasen. Als Sumpfgefäß der Kolonne dient ein Druckreaktionsbehälter aus Stahl (Nenninhalt 5 l, zulässiger Höchstdruck 64 bar) mit Rührer und Mantelheizung.

In den Druckbehälter werden 594 g N,N'-Diphenylharnstoff und 2 075 g n-Butanol eingefüllt. Der Druckbehälter und die Kolonne werden mit Stickstoff gespült und dann verschlossen. Bei abgeschalteter Kolonnenkopfkühlung wird das Gemisch unter Rühren 2 Stunden lang auf 190 °C erhitzt und dann wieder abgekühlt. Dem Gemisch werden 168 g Harnstoff zugesetzt. Anschließend wird die Mischung erneut unter Rühren erhitzt. Dabei wird durch entsprechende Einstellung des Ventils am Kolonnenkopf und der Kühlleistung des Kopfkondensators der sich in der Apparatur einstellende Druck so reguliert, daß die gewünschte Reaktionstemperatur gerade erreicht werden kann. Das Gemisch wird auf 200°C erhitzt und 5,5 Stunden auf 200°C gehalten. Dabei wird Ammoniak freigesetzt, das durch Rektifikation in der Kolonne von mit verdampfenden Substanzen befreit wird und gasförmig am Kolonnenkopf praktisch rein entnommen wird.

Nach Beendigung der Reaktion wird das Reaktionsgemisch abgekühlt und nach dem Entspannen der Apparatur entnommen. Die Lösung wird durch Hochdruckflüssigkeitschromatographie mit Hilfe einer Eichung mit einer authentischen Substanz analysiert. Es werden 831 g N-Phenylcarbamidsäure-n-butylester ausgewiesen, was einer Ausbeute von 77% der Theorie entspricht.

**Beispiel 2**

Entsprechend Beispiel 1 werden in der beschriebenen Druckapparatur 573 g N,N'-Diphenylharnstoff und 2 080 g Ethanol (ca. 96%ig) bei geschlossener Apparatur 1,5 Stunden bei 190°C umgesetzt, dann abgekühlt, mit 162 g Harnstoff und 5,8 g Zinkoctoat versetzt und unter Ammoniak-Entnahme 5,5 Stunden bei 200°C umgesetzt. Nach dem Abkühlen und Entspannen der Apparatur wird das Reaktionsgemisch entnommen, filtriert und fraktioniert destilliert. Bei Normaldruck wird überschüssiger Alkohol abgetrennt. Bei 0,2 mbar destillieren dann als Hauptlauf 783 g (87,8% der Theorie) N-Phenyl-carbamidsäure-ethylester über, die zu Kristallen vom Schmelzpunkt 49-51°C erstarren.

**Beispiel 3**

In der im Beispiel 1 beschriebenen Druckapparatur werden 637 g N,N'-Diphenylharnstoff, 267 g Ethylcarbamat und 1 880 g Ethanol (ca. 96%ig) unter Ammoniak-Entnahme 8,5 Stunden bei 200°C umgesetzt. Nach dem Abkühlen und Entspannen der Apparatur wird das Reaktionsgemisch entnommen, filtriert und fraktioniert destilliert. Zunächst wird bei Normaldruck überschüssiger Alkohol abgetrennt. Dann wird das Gemisch bei 0,2 mbar destilliert, wobei als Hauptlauf 766 g (77,3% der Theorie) N-Phenylcarbamidsäureethylester erhalten werden, die zu Kristallen vom Schmelzpunkt 48-51°C erstarren.

**Beispiel 4**

In der im Beispiel 1 beschriebenen Druckapparatur werden 673 g N,N'-Di-(3-methylphenyl)-harnstoff. 147 g Methylcarbamat, 475 g Methanol und 1 500 g o-Xylol unter Ammoniak-Entnahme 2 Stunden bei 200°C umgesetzt. Dann wird das Gemisch abgekühlt, mit 50 g Harnstoff versetzt und erneut unter Ammoniak-Entnahme 6,0 Stunden bei 200°C umgesetzt. Nach dem Abkühlen und Entspannen der Apparatur wird das Gemisch entnommen, filtriert und flüssigkeitschromatographisch (HPLC) untersucht. Nach Eichung mit einer authentischen Substanz wird eine Ausbeute von 660 g (71% der Theorie) N-(3-Methylphenyl)-carbamidsäure-methylester ermittelt.

**Beispiel 5**

In die im Beispiel 1 beschriebene Druckapparatur werden 524 g eines Polyharnstoff-Gemisches aus 2,4-Diamino-toluol mit Aminotolyl-Endgruppen (mittleres Molgewicht: 1 500) eingefüllt. Dann werden 352 g Ethylcarbamat, 1 200 g Ethanol (ca. 96%ig), 900 g o-Xylol und 5,2 g Zinkoctoat dazugegeben. Das Gemisch wird gerührt und unter Ammoniak-Entnahme 6,5 Stunden bei 200°C umgesetzt. Nach dem Abkühlen und Entspannen der Apparatur wird das Reaktionsgemisch entnommen, filtriert und flüssigkeitschromatographisch (HPLC) analysiert. Nach Eichung mit einer authentischen Substanz wird eine Ausbeute von 716 g (75% der Theorie) 2,4-Bis(ethoxycarbonylamino)-toluol ermittelt.

**Beispiel 6**

In die im Beispiel 1 beschriebene Druckapparatur werden 299 g eines Polyharnstoff-Gemisches aus 4,4'-Diaminodiphenylmethan mit 4-(p-Aminobenzyl)-phenyl-Endgruppen (mittleres Molgewicht: 1 140) eingefüllt. Dann werden 167 g Isopropylcarbamat, 393 g Isopropanol und 2 300 g o-Dichlorbenzol dazugegeben. Das Gemisch wird unter Ammoniak-Entnahme 8,5 Stunden bei 200 °C umgesetzt und dann abgekühlt. Nach dem Entspannen der Apparatur wird das Reaktionsgemisch entnommen, filtriert und durch Hochdruckflüssigkeitschromatographie analysiert. Nach Eichung mit einer authentischen Substanz wird eine Ausbeute von 340 g (68% der Theorie) 4,4'-Bis-(isopropoxycarbonylamino)-diphe-nylmethan ermittelt.

**Beispiel 7**

88 g N,N'-Dimethylharnstoff (1 Mol), 60 g Harnstoff (1 Mol), 250 g Cyclohexanol (2,5 Mol) und 3 ml Zinkoctoat werden gemischt und auf 145°C erwärmt, wobei eine $NH_3$-Entwicklung einsetzt. Innerhalb von 5 Stunden wird die Temperatur auf 195°C gesteigert und weitere 2 Stunden bei dieser Temperatur gehalten.

Nach beendeter Reaktion wird überschüssiges Cyclohexanol abgezogen, der Rückstand an der Ölpumpe destilliert, das Reaktionsprodukte durch IR und KR identifiziert. Ausbeute: 245 g N-Methyl-O-cyclohexylurethan (= 78% der Theorie).

**Beispiel 8**

71,2 g Carbamidsäureethylester (0,8 Mol), 171,6 g der Verbindung

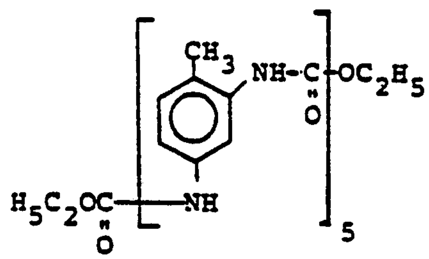

(0,2 Mol), 280 g Cyclohexanol (2,8 Mol) und 5 ml Zinkoctoat werden auf Rückflußtemperatur erwärmt. Es findet nur eine geringe $NH_3$-Entwicklung statt. Nach Zugabe von 113 g Caprolactam (1 Mol) beginnt bei 160°C die $NH_3$-Entwicklung und Alkohol-Abspaltung. Die Innentemperatur wird innerhalb 3 Stunden auf 200°C erhöht und 10 Stunden bei dieser Temperatur gehalten.

Danach werden bei 0,26 mbar das Caprolactam und noch vorhandenes Cyclohexanol abdestilliert.

Nach IR und NMR-spektroskopischem Befund besteht der Rückstand aus 2,4-Bis-(cyclohexoxycarbonylamino)-toluol 254 g = 68% der Theorie).

**Beispiel 9**

64 g der Verbindung

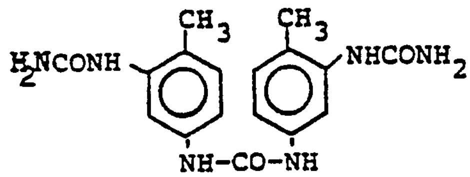

(0,18 Mol), 10,8 g Harnstoff (0,18 Mol), 100 g Cyclohexanol und 113 g Caprolactam (1 Mol) werden mit 2 ml Zinkoctoat versetzt und unter $NH_3$-Entwicklung innerhalb einer Stunde auf 200°C erhitzt und weitere drei Stunden bei dieser Temperatur gehalten. Nach dieser Zeit ist die $NH_3$-Entwicklung beendet. Nach dem Erkalten werden Caprolactam und nicht umgesetztes Cyclohexanol bei 0,26 mbar abdestilliert. Der Rückstand wird durch IR und KR identifiziert, eine Probe umkristallisiert.

Ausbeute; 109 g 2.4-Bis-(cyclohexoxycarbonylamino)-toluol (= 81% der Theorie).

Fp: 155-156°C (aus Toluol).

**Beispiel 10**

89 g Carbamidsäureethylester (1 Mol), 196 g N,N'-(3,3'-Di-dibenzofuranyl)-harnstoff (0,5 Mol), 195 g 2-Ethyl-n-hexanol (1,5 Mol) werden mit 2 g Zinkacetat versetzt und als Lösungsmittel 100 g Caprolactam zugegeben. Die Reaktionsmischung wird innerhalb von 3 Stunden auf 200°C erhitzt. Der entstehende Ammoniak und der Ethylalkohol werden über einen dampfbeheizten Kühler ausgetrieben. Es wird weitere sechs Stunden bei 200°C erhitzt. Nach beendeter Umsetzung werden Caprolactam und restliches 2-Ethyl-n-hexanol im Ölpumpenvakuum abdestilliert.

Der Rückstand wird mit Ethanol aufgekocht, kalt von den unlöslichen Bestandteilen abfiltriert und das Filtrat eingeengt. Der so erhaltene Rückstand wurde durch IR und KR identifiziert.

Ausbeute: 244 g N-(3-Dibenzofuranyl)-O-(2-ethylhexyl)-urethan (= 72% der Theorie).

**Beispiel 11**

105 g N,N'-Diisobutylharnstoff (0,5 Mol), 37 g Harnstoff (0,5 Mol) und 160 g Cyclohexanol (1,6 Mol) werden in Gegenwart von 3 g Kobelanaphthenat innerhalb von 4 Stunden auf 200 °C erwärmt und danach drei weitere Stunden bei dieser Temperatur gelassen; die $NH_3$-Entwicklung ist beendet.

Noch vorhandenes Cyclohexanol wird im Wasserstrahlvakuum entfernt, die Hauptmenge im Hochvakuum destilliert.

Ausbeute: 222 g N-Isobutyl-O-cyclohexylurethan (= 93% der Theorie).

Sdp: 98-192°C bei 0,4 mbar.

**Beispiel 12**

200,6 g N,N'-Dihexylharnstoff (0,88 Mol), 43 g Harnstoff (0,88 Mol) und 210 g Cyclohexanol (2,1 Mol) werden mit 3 g Zinkcyanid gemischt und auf 150°C erwärmt, wobei eine $NH_3$-Entwicklung einsetzt. Die Innentemperatur wird auf 200°C gesteigert und 4 Stunden bei dieser Temperatur behalten. Danach wird Cyclohexanol bei 15 mbar abdestilliert und der Rückstand im Ölpumpenvakuum fraktioniert.

Ausbeute: 368 g N-(n-Hexyl)-O-cyclohexylurethan (= 92% der Theorie).

Sdp: 123-125°C bei 0,06 mbar.

**Beispiel 13**

202 g N,N'-Dicyclohexylharnstoff (0,9 Mol), 55 g Harnstoff (0,9 Mol) und 180 g Cyclohexanol (1,8 Mol) werden mit 3 ml Zinkoctoat versetzt und innerhalb von 3 Stunden auf 200°C erhitzt. Nach 3 Stunden bei dieser Temperatur läßt die $NH_3$-Entwicklung nach und die Reaktion wird beendet. Der Rückstand wird destilliert.

Ausbeute: 415 g N-Cyclohexyl-O-cyclohexylurethan (= 84% der Theorie).

Sdp: 142-145°C bei 0,13 mbar.

**Beispiel 14**

30 g (0,5 Mol) Harnstoff, 106 g (0,5 Mol) N,N'-Diphenylharnstoff und 305 g (2,5 mol) β-Phenylethanol wurden mit 1 g Zinkcyanid 4 Stunden am Rückfluß erhitzt. Mit Hilfe der Hochdruckflüssigkeitschromatographie wurde, die Ausbeute an N-Phenyl-O-β-phenylethylurethan zu 92% der Theorie bestimmt. Nach

Aufarbeitung konnten 212 g = 88% der Theorie des Urethans vom Schmelzpunkt 78°C (Waschbenzin) isoliert werden.

**Beispiel 15**

106 g (1 Mol) N,N'-Diphenylharnstoff, 300 g (3 Mol) Cyclohexanol, 51,5 g (0,5 Mol) Biuret und 0,7 g Zinkoctoat wurden 10 Stunden am Rückfluß erhitzt. Die Ausbeute an N-Phenyl-O-cyclohexyl-urethan wurde hochdruckflüssigkeitschromatographisch zu 96% der Theorie bestimmt.

**Beispiel 16**

106 g (0,5 Mol) N,N'-Diphenylharnstoff, 50 g (0,5 Mol) Cyclohexanol und 71,5 g (0,5 Mol) Carbamidsäurecyclohexylester wurden mit 0,8 g Zinkoctoat 4 Stunden am Rückfluß erhitzt wobei die Temperatur auf 200°C anstieg. Nach Zugabe von weiteren 0,3 Mol Cyclohexanol wurde noch 5 Stunden bei 200°C gehalten. Mit Hilfe der Hochdruckflüssigkeitschromatographie wurde analysiert und festgestellt, daß sich 80% der Theorie an N-Phenyl-O-cyclohexyl-urethan gebildet hatten.

**Beispiel 17**

424 g (2 Mol) N,N'-Diphenylharnstoff und 120 g (2 Mol) Harnstoff wurden in 800 g (8 Mol) Cyclohexanol 12 Stunden bei Rückflußtemperatur gerührt. Das Cyclohexanol wurde abdestilliert und der Rückstand aus Waschbenzin umkristallisiert. Es wurden 90% der Theorie an N-Phenyl-O-cyclohexyl-urethan vom Fp. 74-76°C erhalten.

Die Reaktion wurde mit 1 g Nickelchlorid und 600 g (6 Mol) Cyclohexanol durchgeführt. Die Ausbeute an O-Cyclohexyl-N-phenylurethan betrug nach 8 Stunden Reaktionsdauer 91% der Theorie.

**Beispiel 18**

60 g (1 Mol) Harnstoff, 106 g (0,5 Mol) N,N'-Diphenylharnstoff, 68 g (0,5 Mol) Carbamidsäurephenylester und 300 g (3 Mol) Cyclohexanol wurden am auf 90°C beheizten Kühler am Rückfluß erhitzt, so daß das entstandene Ethanol laufend abdestilliert wurde. Nach 4 Stunden waren 200°C erreicht, die weitere 6 Stunden beibehalten wurden. Anschließend wurde im Hochvakuum (0,2 Torr, 120°C) eingeengt und der Rückstand mit Hilfe der Hochdruckflüssigkeitschromatographie analysiert. Es hatten sich 93% der Theorie N-Phenyl-O-cyclohexyl-urethan gebildet.

**Beispiel 19**

In einem 2 l-Dreihalskolben mit Rührer, Kontaktthermometer und Rückflußkühler wurden 600 g (6 Mol) Cyclohexanol bei 150-160°C vorgelegt. Im Verlauf von 2 Stunden wurden 392 g (2 Mol Harnstoffgruppen) eines Polyharnstoffs portionsweise eingetragen, der durch Umsetzung von 1-Amino-3.3.5-trimethyl-5-aminomethyl-cyclohexan mit Harnstoff in Dichlorbenzol synthetisiert worden war (mittleres Molekulargewicht ca 1 200). Nach Abkühlen auf 120°C wurden 120 g (2 mol) Harnstoff und 5 g Kupferacetat zugefügt. Im Verlauf von 6 Stunden wurde die Temperatur unter Abdestillieren von 100 g (1 Mol) Cyclohexanol allmählich auf 210°C gesteigert. Dabei entstanden 3,8 Mol Ammoniak (95% der Theorie).

Nun wurde im Vakuum überschüssiges und nicht umgesetztes Cyclohexanol abdestilliert. Es blieben 820 g eines gelben Harzes zurück, welches zu 76% aus 1-(Cyclohexoxycarbonylamino)-3,3,5-trimethyl-5-(cyclohexoxycarbonylaminomethyl)-cyclohexan bestand, wie hochdruckflüssigkeitschromatographisch nachgewiesen wurde.

Ausbeute: 74% der Theorie.

**Patentansprüche**

1. Verfahren zur Herstellung von Urethanen der allgemeinen Formel

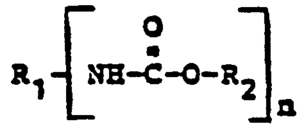

in welcher

$R_1$ für einen gegebenenfalls substituierten aliphatischen Kohlenwasserstoff rest mit 1-18 Kohlenstoffatomen, einen gegebenenfalls substituierten cycloaliphatischen Kohlenwasserstoffrest mit 3 bis 18 Kohlenstoffatomen, einen gegebenenfalls substituierten aromatischen Kohlenwasserstoffrest mit 6-15 Kohlenstoffatomen, einen gegebenenfalls substituierten araliphatischen Kohlenwasserstoffrest mit 7 bis 14 Kohlenstoffatomen, oder für einen gegebenenfalls substituierten 5- oder 6-gliedrigen heterocyclischen Rest steht, der zusätzlich noch mit einem Benzolring anelliert sein kann,

$R_2$ für einen gegebenenfalls substituierten Alkylrest mit 1 bis 20 Kohlenstoffatomen, einen gegebenenfalls substituierten Cycloalkylrest mit 3 bis 16 Kohlenstoffatomen und einen gegebenenfalls substituierten Aralkylrest mit 7 bis 14 Kohlenstoffatomen und

n eine ganze Zahl von 1 bis 3 bedeuten,

wobei im Falle von n = 2 oder 3 zwischen 2 mit dem Rest $R_1$ verknüpften Urethangruppen mindestens 2 Kohlenstoffatome angeordnet sind, durch Umsetzung als Ausgangsmaterialien von

a) gegebenenfalls Urethan. oder Aminoendgruppen aufweisenden N-mono- oder N,N'-disubstituierten Harnstoffen oder linearen Polyharnstoffen eines maximalen Molekulargewichts von 2 000, deren Harnstoff-, Urethan- bzw. Aminogruppen über Kohlenwasserstoffreste miteinander verknüpft sind, bzw. deren Harnstoffgruppen mit Kohlenwasserstoffresten substituiert sind, die die Bedeutung von $R_1$ haben, deren gegebenenfalls vorliegende endständige Urethangruppan am Sauerstoffatom eines Substituenten aufweisen, dessen Bedeutung der Definition von $R_2$ entspricht, und deren Gesamtgehalt an Harnstoffgruppen -NH-CO-NH- und Urethangruppe -NH-CO-O einem Gehalt an für beide Gruppen charakteristischen Struktureinheiten -NH-CO zwischen 5 und 58 Gew.-% entspricht, mit

b) Alkoholen der Formel

$R_2$-OH

im Temperaturbereich von 120 bis 350° C, dadurch gekennzeichnet, daß man als weitere Reaktionspartner

c) Urethane der Formel

$R_3$-O-CO-$NH_2$

in welcher,

$R_3$ entweder der Bedeutung von $R_2$ entspricht, wobei $R_2$ und $R_3$ gleiche oder verschiedene Reste bedeuten, oder für einen gegebenenfalls Chlor, oder $C_1$-$C_4$-Alkyl-substituierten. aromatischen Kohlenwasserstoffrest mit insgesamt 6-15-Kohlenstoffatomen steht, und/oder

d) Harnstoff und/oder Polyurete der Formel

$H_2N$-$(CO$-$NH)_m$-H

in einer Menge von mindestens 10 Gew.-% der Komponenten

c) und/oder d), bezogen auf die Komponente a) mitverwendet, wobei

m für eine ganze Zahl von 1 bis 4 steht.

2. Verfahren gemäß Anspruch 1, dadurch gekennzeichnet, daß man die Umsetzung in Gegenwart von e) Veresterungskatalysatoren für Carbonsäuren als Katalysatorren durchführt.

3. Verfahren gemäß Anspruch 1 und 2, dadurch gekennzeichnet daß man die Umsetzung i Gegenwart von polaren Lösungsmitteln durchführt.

4. Verfahren gemäß Anspruch 3, dadurch gekennzeichnet. daß man die Umsetzung in Gegenwart von ε-Caprolactam als Lösungsmittel durchführt.

**Claims**

1. Process for the production of urethanes of the general formula

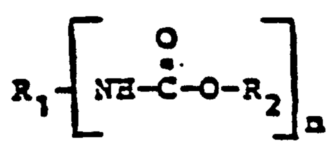

in which

$R_1$ represents an optionally substituted aliphatic hydrocarbon radical with 1-18 carbon atoms, an optionally substituted cycloaliphatic hydrocarbon radical with 3 to 18 carbon atoms, an optionally substituted aromatic

hydrocarbon radical with 6 -15 carbon atoms, an optionally substituted araliphatic hydrocardon radical bith 7 to 14 carbon atoms, or an optionally substituted 5- or 6-membered heterocyclic radical hich can additionally be fused to a benzene ring, $R_2$ represents an optionally substituted alkyl radical with 1 to 20 carbon atoms, an optionally substituted cycloalkyl radical with 3 to 16 carbon atoms and an optionally substituted aralkyl radical with 7 to 14 carbon atoms and

n denotes an integer from 1 to 3,

wherein in the case where n = 2 or 3 at least 2 carbon atoms are arranged between 2 urethane groups attached to the radical $R_1$,

by reacting; as starting materials,

a) N-mono- or N,N'-disubstituted ureas or linear polyureas of a maximum molecular weight of 2,000, which optionally contain urethane or amino end groups and whose urea, urethane or amino groups are linked to one another via hydrocarbon radicals, or whose urea groups are substituted by hydrocarbon radicals having the meaning of $R_1$, whose optionally present terminal urethane groups have a substituent on the oxygen atom whose meaning corresponds to the definition of $R_2$, and whose total content of urea groups -NH-CO-NH- and urethane group -NH-CO-O- corresponds to a content of structural units -NH-CO characteristic of both groups of between 5 and 58% by weight,

with

b) alcohols of the formula

$R_2$-OH

in the temperature range of 120 to 350° C, characterised in that c) urethanes of the formula

$R_3$-O-CO-$NH_2$

in which

$R_3$ either corresponds to the meaning of $R_2$, $R_2$ and $R_3$ denoting identical or different radicals, or represents an optionally chlorine- or $C_1$-$C_4$-alkyl-substituted, aromatic hydrocarbon radical bith a total of 6-15 carbon atoms, and/or

d) urea and/or polyurets of the formula,

$H_2N$--$(CO-NH)_m$-H

are also used as further reactants, in a quantity of at least 10% by weight of components c) and/or d), based on component a), wherein m represents an integer from 1 to 4.

2. Process according to Claim 1, characterised in that the reaction is carried out in the presence of e) esterification catalysts for carboxylic acids as the catalysts.

3. Process according to Claim 1 and 2, characterised in that the reaction is carried out in the presence of polar solvents.

4. Process according to Claim 3, characterised in that the reaction is carried out in the presence of ε-caprolactam as the solvent.

**Revendications**

1. Procédé de production d'uréthannes de formule générale:

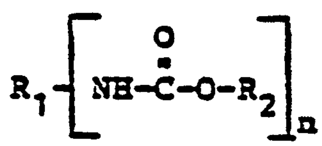

dans laquelle

$R_1$ désigne un reste d'hydrocarbure aliphatique ayant 1 à 18 atomes de carbone éventuellement substitué, un reste d'hydrocarbure cycloaliphatique ayant 3 à 18 atomes de carbone éventuellement substitué, un reste d'hydrocarbure aromatique ayant 6 à 15 atomes de carbone éventuellement substitué, un reste d'hydrocarbure araliphatique ayant 7 à 14 atomes de carbone éventuellement substitué ou un reste hétérocyclique pentagonal ou hexagonal éventuellement substitué qui peut en outre être condensé avec un noyau benzénique,

$R_2$ est un reste alkyle ayant 1 à 20 atomes de carbone éventuellement substitué, un reste cycloalkyle ayant 3 à 16 atomes de carbone éventuellement substitué et un reste aralkyle ayant 7 à 14 atomes de carbone éventuellement substitué et

n est un nombre entier de 1 à 3,

au moins deux atomes de carbone étant disposés entre deux groupes uréthanne liés avec le reste $R_1$ au cas où n est égal à 2 ou à 3,

par réaction, en tant que matières de depart

a) d'urées N-mono- ou N,N'-disubstituées ou de polyurées linéaires portant éventuellement des groupes uréthanne ou amino terminaux, d'un poids moléculaire maximal de 2000, dont les groupes urée, uréthanne ou amino sont liés les uns avec les autres par l'intermédiaire de restes hydrocarbonés, ou dont les groupes urée sont substitués avec des restes hydrocarbonés qui ont la définition de $R_1$, dont des groupes uréthanne terminaux éventuellement présents portent sur l'atome d'oxygène un substituant dont la définition correspond

à celle de $R_2$, et dont la teneur totale en groupes urée -NH-CO-NH -et en groupes uréthanne -NH-CO-O- correspond à une teneur en motifs structuraux -NH-CO caractéristiques des deux groupes comprise entre 5 et 58 % en poids, avec

b) des alcools de formule $R_2$-OH dans une plage de températures de 120 à 350°C, caractérisé en ce qu'on utilise simultanément comme autres partenaires réactionnels

c) des uréthannes de formule

$R_3$-O-CO-$NH_2$

dans laquelle

$R_3$ correspond à la définition de $R_2$, auquel cas $R_2$ et $R_3$ désignent des restes égaux ou différents, ou bien représente un reste hydrocarboné aromatique totalisant 6 à 15 atomes de carbone, éventuellement substitué par du chlore ou par un groupe alkyle en $C_1$ à $C_4$, et/ou

d) de l'urée et/ou des polyurets de formule

$H_2N$-(CO-NH$)_m$-H

en une quantité d'au moins 10 % en poids des composants c) et/ou d), par rapport au composant a)

m désignant un nombre entier de 1 à 4.

2. Procédé suivant la revendication 1, caractérisé en ce qu'on conduit la réaction en présence de catalyseurs qui sont e) des catalyseurs d'estérification pour acides carboxyliques.

3. Procédé suivant les revendications 1 et 2, caractérisé en ce qu'on conduit la réaction en présence de solvants polaires.

4. Procédé suivant la revendication 3, caractérisé en ce qu'on conduit la réaction en présence d'ε-caprolactame comme solvant.